# FASCICULE DE BREVET EUROPEEN

(11) **EP 4 129 231 B1**
(45) Date de publication et mention de la délivrance du brevet: **03.06.2026**
(21) Numéro de dépôt: 22190668.8
(22) Date de dépôt: 21.04.2017
(51) Int. Cl.: A61C 7/00, G16H 50/50, G16H 50/70, G16H 30/40

(54) **SYSTÈME DE CONTRÔLE D'UNE SITUATION DENTAIRE**
SYSTEM ZUR KONTROLLE EINER ZAHNLAGE
SYSTEM FOR MONITORING A DENTAL SITUATION

(30) Priorité: 22.04.2016 FR 1653589
(43) Date de publication de la demande: 08.02.2023
(62) Demande divisionnaire de: 17718909.9
(73) Titulaire: Dental Monitoring, 75017 Paris (FR)
(72) Inventeur: SALAH, Philippe, 75020 Paris (FR); AYACHE, William, 92200 NEUILLY SUR SEINE (FR); GHYSELINCK, Guillaume, 59169 CANTIN (FR); DEBRAUX, Laurent, 75020 Paris (FR); ROISIN, Louis Charles, 75016 Paris (FR); PELLISSARD, Thomas, 92110 Clichy (FR)
(74) Mandataire: Novagraaf Group

(56) Documents cités:
- WO-A1-2008/149221
- WO-A1-2016/135549
- CA-A1- 2 820 539
- US-A1- 2004 197 727
- US-A1- 2010 151 404

## Description

### Domaine technique

La présente invention concerne un système de contrôle de la situation dentaire future d'un patient.

### Etat de la technique

Classiquement, au début d'un traitement orthodontique, l'orthodontiste détermine le positionnement des dents qu'il souhaite obtenir à l'issue du traitement, dit "set-up final". Le set-up final peut être défini au moyen d'une empreinte ou à partir d'un scan tridimensionnel des dents du patient. L'orthodontiste fabrique alors, en conséquence, un appareil orthodontique adapté à ce traitement.

L'appareil orthodontique peut être classiquement un appareil à attaches comportant un arc orthodontique métallique attaché aux dents.

Alternativement, l'appareil orthodontique peut être une gouttière (*« aligner »* en anglais). Une gouttière orthodontique se présente classiquement sous la forme un appareil monobloc amovible, classiquement en un matériau polymère transparent, qui comporte une goulotte conformée pour que plusieurs dents d'une arcade, généralement toutes les dents d'une arcade, puissent y être logées. La forme de la goulotte est adaptée pour maintenir en position la gouttière sur les dents, tout en exerçant une action de correction de la position de certaines dents.

Le traitement au moyen de gouttières est avantageusement moins contraignant pour le patient. En particulier, le nombre de rendez-vous chez l'orthodontiste est limité. En outre, la douleur est plus faible qu'avec un arc orthodontique métallique attaché aux dents.

Le marché des gouttières orthodontiques est donc en croissance.

Dans le cas où l'appareil orthodontique est une gouttière, on détermine classiquement, au début du traitement, les formes que doivent prendre les différentes gouttières à différents instants du traitement. Le positionnement souhaité des dents chacun de ces instants est appelé « set-up intermédiaire ».

L'orthodontiste fait ensuite fabriquer l'ensemble des gouttières correspondantes. Le nombre de gouttières est classiquement d'une vingtaine. Aux instants prédéterminés, le patient change de gouttière.

Quel que soit l'appareil orthodontique mis en œuvre, à intervalles réguliers, le patient se déplace chez l'orthodontiste pour un contrôle visuel. En fonction de son diagnostic, l'orthodontiste modifie éventuellement l'appareil orthodontique.

Par exemple, dans le cas où l'appareil orthodontique est un appareil comportant un arc orthodontique métallique attaché aux dents, l'orthodontiste peut modifier la tension exercée par l'arc orthodontique. Le cas échéant, il peut également faire fabriquer un nouvel appareil orthodontique mieux ajusté.

Dans le cas où l'appareil orthodontique est une gouttière, l'orthodontiste peut effectuer une nouvelle empreinte des dents, ou, de manière équivalente, un nouveau scan tridimensionnel des dents, puis commander une nouvelle série de gouttières. On considère qu'en moyenne, le nombre de gouttières finalement fabriquées est d'environ 45, au lieu des 20 gouttières classiquement prévues au début du traitement.

La nécessité de devoir se déplacer chez l'orthodontiste pour un contrôle est une contrainte pour le patient. La multiplication des contrôles peut également entamer la confiance du patient en son orthodontiste. Enfin, il en résulte un coût supplémentaire.

Il existe donc un besoin pour limiter le nombre de contrôles chez l'orthodontiste.

Par ailleurs, il existe un besoin permanent pour accélérer les traitements orthodontiques. Par ailleurs, hors de tout traitement orthodontique, il existe un besoin pour mieux anticiper l'évolution de la position des dents des patients, en particulier lors de la croissance des dents des enfants ou du vieillissement des personnes âgées. Chez ces dernières, le vieillissement conduit en particulier à un avancement des dents.

Un but de l'invention est de répondre, au moins partiellement, à ces besoins.

Le document US2010151404A1 montre un système pour détecter des écarts par rapport à un plan de traitement orthodontique. Un graphique représente selon l'axe x le mouvement prévu et selon l'axe y le mouvement réel.

### Résumé de l'invention

L'invention a pour objet un système de contrôle de la situation dentaire future d'un patient, dit « patient actuel », selon l'une des revendications 1 à 9.

La description divulgue également un procédé de prédiction d'une situation dentaire future pour un patient, dit « patient actuel », en particulier pour un patient actuel portant un appareil orthodontique, dit « appareil orthodontique actuel », destiné à corriger une malposition de ses dents, ledit procédé de prédiction comportant les étapes suivantes :
1) acquisition de données, dites « données historiques », relatives à des situations dentaires passées, dites « situations dentaires historiques », vécues chacune, à un instant, dit « instant historique », par un patient, dit « patient historique », le patient historique pouvant en particulier suivre un traitement orthodontique dit « traitement orthodontique historique », au cours duquel il est muni d'un appareil orthodontique, dit « appareil orthodontique historique »,
   l'ensemble des données historiques relatives à une situation dentaire historique comportant au moins:
   - ledit instant historique ;
   - des valeurs de paramètres de contexte audit instant historique, les paramètres de contexte comportant :
      - de préférence, si le patient historique est muni d'un appareil orthodontique historique,
         - des paramètres dudit appareil orthodontique historique, en particulier relatifs à la classe et/ou à la conformation de l'appareil orthodontique historique ;
         - de préférence, des paramètres sur l'environnement du traitement orthodontique historique auquel la situation dentaire historique appartient, comme un coefficient de douleur et/ou un coût et/ou une durée et/ou un nombre de rendez-vous chez l'orthodontiste et/ou une probabilité de succès associé(s) audit traitement orthodontique historique;
      - des paramètres de positionnement de dents dudit patient historique ;
      - de préférence d'autres paramètres anatomiques que les paramètres de positionnement de dents, comme l'agencement et/ou la structure de tissus osseux (notamment des mâchoires) et/ou de tissus alvéodentaires et/ou de tissus mous (notamment les gencives et/ou les freins et/ou la langue et/ou les joues), du patient historique ;
      - de préférence, des paramètres fonctionnels du patient historique, en particulier des paramètres neurofonctionnels, comme la facilité à respirer, à déglutir ou à fermer la bouche ;
      - de préférence, l'âge et/ou le sexe et/ou un identifiant dudit patient historique ;
2) acquisition, à un instant, dit « instant actuel », de données relatives à une situation dentaire vécue par ledit patient actuel, dite « situation dentaire actuelle », l'ensemble des données relatives à ladite situation dentaire actuelle, dites « données actuelles », comportant au moins:
   - de préférence, ledit instant actuel ;
   - des valeurs de paramètres de contexte audit instant actuel, les paramètres de contexte comportant :
      - de préférence, si le patient actuel est muni d'un appareil orthodontique actuel,
         - des paramètres dudit appareil orthodontique actuel, en particulier relatifs à la classe et/ou à la conformation de l'appareil orthodontique actuel ;
         - de préférence, des paramètres sur l'environnement du traitement orthodontique actuel auquel la situation dentaire actuelle appartient, dit « traitement orthodontique actuel », comme un coefficient de douleur et/ou un coût et/ou une durée et/ou un nombre de rendez-vous chez l'orthodontiste et/ou une probabilité de succès associé(s) audit traitement orthodontique actuel ;
      - des paramètres de positionnement de dents dudit patient actuel ;
      - de préférence d'autres paramètres anatomiques que les paramètres de positionnement de dents, comme l'agencement et/ou la structure de tissus osseux (notamment des mâchoires) et/ou de tissus alvéodentaires et/ou de tissus mous (notamment les gencives et/ou les freins et/ou la langue et/ou les joues), du patient actuel ;
      - de préférence, des paramètres fonctionnels du patient actuel, en particulier des paramètres neurofonctionnels, comme la facilité à respirer, à déglutir ou à fermer la bouche ;
      - de préférence, l'âge et/ou le sexe et/ou un identifiant dudit patient actuel ;
3) analyse statistique desdites données historiques et desdites données actuelles, de manière à prédire, à au moins un instant futur, au moins une situation dentaire future pour le patient actuel ;
4) en fonction de ladite situation dentaire future, évaluation de l'intérêt d'un traitement orthodontique pour la patient actuel ou, si le patient actuel est muni d'un appareil orthodontique actuel, réévaluation du traitement orthodontique actuel, de préférence par un orthodontiste, et, en fonction de ladite réévaluation, modification éventuelle du traitement orthodontique du patient actuel, par exemple modification ou changement de l'appareil orthodontique actuel et/ou modification d'un planning de rendez-vous avec un orthodontiste.

La capacité de prédiction de situations dentaires futures constitue un avantage considérable par rapport à la situation antérieure à l'invention.

De manière générale, il devient en effet possible de prédire une évolution de la position des dents, dans le cadre d'un traitement orthodontique ou non. Par exemple, il devient possible de prévoir comment les dents d'un enfant vont se déplacer lors de leur croissance, ce qui permet d'agir très tôt pour corriger une situation défavorable.

De même, les dents ont tendance à se déplacer, notamment lorsque le patient vieillit. Grâce à l'invention, il devient possible de prévoir ce déplacement, ce qui permet d'agir très tôt pour corriger une situation défavorable.

La capacité de prédiction de situations dentaires futures constitue en particulier un avantage considérable lorsque le patient actuel suit un traitement orthodontique.

En effet, jusqu'à la présente invention, lors d'un contrôle, l'orthodontiste ne pouvait percevoir que les anomalies les plus visibles, par exemple un décollement important de la gouttière portée dans certaines zones. Sa capacité d'anticipation était donc limitée. En outre, l'orthodontiste pouvait mal interpréter une situation. Par exemple, il pouvait considérer qu'un décollement d'une gouttière correspondait à une anomalie, alors que ce décollement n'était que provisoire, ou allait rester limité.

Avantageusement, la prédiction réalisée par le procédé selon la description lui permet d'appréhender une situation future que la seule observation visuelle ne permet pas de concevoir. Il peut donc, par exemple, choisir de ne pas modifier l'appareil orthodontique actuel alors même qu'il perçoit un décollement. Réciproquement, il peut modifier l'appareil orthodontique actuel alors même qu'il ne perçoit pas de décollement, ou un décollement très faible.

Comme on le verra plus en détail dans la suite de la description, l'efficacité du traitement orthodontique en est considérablement améliorée.

L'appareil orthodontique actuel peut être une gouttière. Un procédé selon la description est particulièrement bien adapté pour déterminer les instants les plus adaptés pour changer la gouttière portée par un patient actuel.

A l'étape 3), de préférence, on détermine,
- pour au moins une, de préférence pour chaque dite situation dentaire future, et/ou
- pour au moins un, de préférence pour chaque dit instant futur,

- si le patient actuel est muni d'un appareil orthodontique actuel, des valeurs, audit instant futur, de paramètres dudit appareil orthodontique actuel ; et/ou
- des valeurs de paramètres de positionnement des dents du patient actuel audit instant futur (en particulier à un instant futur objectif) ; et/ou
- de préférence, un écart de la valeur d'un ou plusieurs paramètres de positionnement des dents du patient actuel avec la valeur dudit ou desdits paramètres de positionnement dans une situation dentaire constituant un objectif audit instant futur ; et/ou
- un coût pour que ladite situation dentaire future se réalise ; et/ou
- un coefficient de douleur pour que ladite situation dentaire se réalise ; et/ou
- une probabilité que les prédictions relatives aux paramètres de l'appareil dentaire actuel si le patient actuel est muni d'un tel appareil et/ou aux paramètres de positionnement desdites dents dudit patient actuel, et/ou audit coût et/ou audit coefficient de douleur soient conformes à la réalité.

De préférence, à l'étape 3), on détermine plusieurs situations dentaires futures, pour un même instant futur, et/ou au moins une situation dentaire future, pour plusieurs instants futurs différents.

De préférence, on effectue plusieurs dites analyses statistiques, en modifiant à chaque fois ledit instant futur, de manière à prédire des situations dentaires futures jusqu'à un instant futur objectif, par exemple jusqu'à un set-up intermédiaire ou final. Ce cyclage permet de prédire une évolution de la position des dents et/ou un « traitement orthodontique potentiel » jusqu'à l'instant futur objectif.

De préférence, on détermine un ou plusieurs traitements orthodontiques potentiels permettant d'obtenir, à un instant futur objectif, une situation dentaire future objectif déterminée ou une situation dentaire future objectif entrant dans une plage de situations dentaires déterminée.

De préférence, on détermine des premier et deuxième traitements orthodontiques potentiels conduisant, audit instant futur objectif, pour au moins un paramètre de positionnement de dents du patient actuel, à des situations dentaires extrêmes. Les situations « extrêmes » correspondent à des limites minimale et maximale pour ledit paramètre de positionnement, c'est-à-dire à des limites en deçà et au-delà desquelles, respectivement, la situation dentaire audit instant futur objectif est considérée comme non acceptable.

Représentées sur un même graphique, les courbes représentant l'évolution temporelle de la valeur dudit paramètre de positionnement pour ces premier et deuxième traitements orthodontiques potentiels délimitent une surface, appelée « biozone » qui, avantageusement, permet de facilement d'identifier une dérive anormale de positionnement des dents. Il suffit en effet, à un instant quelconque, de mesurer la valeur dudit paramètre de positionnement et de vérifier si, à cet instant, elle entre dans la biozone.

L'instant de départ du ou des traitements orthodontiques potentiels peut être en particulier l'instant actuel ou un instant initial correspondant au début d'un traitement orthodontique actuel avec un appareil orthodontique porté par le patient actuel.

De préférence, on détermine plusieurs traitements orthodontiques potentiels en modifiant à chaque fois au moins une contrainte.

Avantageusement, l'orthodontiste peut ainsi évaluer l'impact d'une modification d'une contrainte. Par exemple, il peut modifier la contrainte du diamètre de l'arc métallique et observer l'effet de cette modification sur le traitement orthodontique potentiel.

De préférence, le procédé comporte opération d'optimisation des contraintes en fonction d'au moins un critère d'optimisation, opération dans laquelle on met en œuvre une succession d'analyses statistiques en modifiant à chaque fois une ou plusieurs desdites contraintes pour un même instant futur, jusqu'à trouver une situation dentaire optimale au regard du critère d'optimisation, suivant au moins une règle d'optimisation.

De préférence, on établit, par la mise en œuvre d'une succession d'analyses statistiques, plusieurs traitements orthodontiques potentiels correspondant à l'application de contraintes différentes, jusqu'à trouver un traitement orthodontique potentiel optimal au regard du critère d'optimisation suivant au moins une règle d'optimisation.

De préférence, le critère d'optimisation est choisi dans le groupe formé par un coefficient de douleur, un coût, un écart avec une valeur souhaitée pour un paramètre de positionnement, une durée, un nombre de rendez-vous chez l'orthodontiste, un nombre de gouttières, une probabilité de succès ou une combinaison de ces critères, chaque critère pouvant être associé au traitement orthodontique actuel ou à une situation dentaire dudit traitement orthodontique actuel, par exemple ladite situation dentaire actuelle ou à un instant futur objectif.

### Définitions

Un « patient actuel » est une personne pour laquelle le procédé selon la description est mis en œuvre pour prédire une situation dentaire future, indépendamment du fait que cette personne présente une malposition des dents ou non. Le patient est dit « actuel » dans un souci de clarté, pour le distinguer d'un patient « historique ».

Par « situation dentaire », on entend une situation relative à une position des dents.

Une « catégorie » de patients regroupe tous les patients en fonction de données physiologiques, par exemple regroupe tous les patients dans une tranche d'âge particulière et/ou du même sexe.

Une « classe » d'appareils orthodontiques définit un ensemble d'appareils orthodontiques comparables. Par exemple, une classe d'appareils orthodontiques peut regrouper tous les appareils orthodontiques qui sont des gouttières actives en un même matériau, ou tous les appareils orthodontiques à attaches pourvus d'un même arc métallique.

Les « paramètres de contexte » sont les paramètres utiles pour évaluer une situation dentaire. Ils comportent notamment les paramètres de positionnement de dents du patient considéré et, le cas échéant, les paramètres de l'appareil orthodontique considéré.

Les "paramètres de l'appareil orthodontique" comprennent des paramètres intrinsèques, comme le(s) matériau(x) qui constitue(nt) ledit appareil orthodontique ou les paramètres de forme au repos, par exemple la forme d'une gouttière ou le diamètre d'un arc orthodontique. Ils comprennent également des paramètres d'application.

Dans un mode de réalisation, les paramètres de l'appareil orthodontique sont des paramètres généraux qui identifient la classe de l'appareil orthodontique.

Un « paramètre de forme » est un paramètre utile pour déterminer la forme d'un appareil orthodontique. Par exemple, *x, y* et z sont des paramètres de forme dans un référentiel *Oxyz* cartésien. Les valeurs de ces paramètres de forme permettent de définir la position d'un point de l'appareil orthodontique dans l'espace, de préférence un point de la surface de l'appareil orthodontique. Un paramètre de forme peut être également un diamètre d'un arc orthodontique, une épaisseur de matière ou une dimension, par exemple.

Un « paramètre d'application » est un paramètre utile pour déterminer la façon dont opère l'appareil orthodontique dans la situation dentaire considérée. La position des points d'attache de l'arc orthodontique sur les dents ou la tension de l'arc orthodontique sont des exemples de paramètres d'application. La durée pendant laquelle l'appareil orthodontique a déjà été porté à l'instant considéré est également un paramètre d'application.

Un « paramètre de positionnement » est un paramètre utile pour déterminer la position d'une dent. Par exemple, l'abscisse, souvent notée x, l'ordonnée, souvent notée *y*, et la cote, souvent notée z, sont des paramètres de positionnement dans un référentiel Oxyz cartésien. La coordonnée radiale, souvent notée *r* ou ρ, et appelée rayon, la coordonnée angulaire, également appelée angle polaire ou azimut, et souvent notée *t* ou *θ*, et la hauteur, souvent notée h, sont des paramètres de positionnement dans un référentiel cylindrique. Les valeurs de ces paramètres de positionnement permettent de définir la position d'un point dans l'espace.

Les paramètres utilisés pour déterminer la forme d'un appareil orthodontique peuvent être identiques ou différents des paramètres utilisés pour déterminer la position des dents. Dans un souci de clarté, ces paramètres ont été désignés différemment, par "paramètres de forme" et "paramètres de positionnement", respectivement.

Les "contraintes" sont des paramètres dont les valeurs ne sont pas libres. On distingue :
- les contraintes "non ajustables", en particulier les paramètres de positionnement des dents du patient actuel à l'instant actuel ou les paramètres relatifs au patient actuel, comme son âge ou son sexe,
- les contraintes "ajustables", pour lesquelles l'orthodontiste ou le patient actuel peuvent fixer des plages de variation, à l'instant actuel et/ou à un ou plusieurs instants futurs.

Une plage de positions acceptables pour les dents du patient actuel à un instant futur, un coût maximal de traitement, un coefficient de douleur maximal au cours du traitement ou un coefficient de douleur moyen pendant le traitement orthodontique actuel sont des exemples de contraintes ajustables.

En particulier lorsqu'on recherche un appareil orthodontique plus adapté au traitement orthodontique, par modification ou remplacement de l'appareil orthodontique actuel, les paramètres de l'appareil orthodontique actuel, par exemple la forme d'une gouttière, peuvent être également des contraintes ajustables.

Les « conditions d'acquisition » d'une image précisent la position et l'orientation dans l'espace d'un appareil d'acquisition d'images relativement aux dents du patient ou à un modèle de dents du patient, et de préférence la calibration de cet appareil d'acquisition d'images. Des conditions d'acquisition sont dites "virtuelles" lorsqu'elles correspondent à une simulation dans laquelle l'appareil d'acquisition serait dans lesdites conditions d'acquisition (positionnement et de préférence calibration théoriques de l'appareil d'acquisition).

Un « set-up » correspond classiquement à un positionnement des dents que le traitement a pour objectif d'atteindre à un instant du traitement, en particulier à la fin du traitement (« set-up final ») ou à une étape intermédiaire prédéterminée du traitement (« set-up intermédiaire »), par exemple à un instant prévu pour changer de gouttière ou modifier la tension de l'arc orthodontique.

La « calibration » d'un appareil d'acquisition est constituée par l'ensemble des valeurs des paramètres de calibration. Un "paramètre de calibration" est un paramètre intrinsèque à l'appareil d'acquisition (à la différence de sa position et de son orientation) dont la valeur influence l'image acquise. De préférence, les paramètres de calibration sont choisis dans le groupe formé par l'ouverture de diaphragme, le temps d'exposition, la distance focale et la sensibilité.

Par "image", on entend une image en deux dimensions, comme une photographie. Une image est formée de pixels.

Une image « acquérable » ("preview") est l'image que l'appareil d'acquisition peut enregistrer à un instant donné. Pour un appareil photo ou un téléphone, c'est l'image qui apparaît sur l'écran lorsque l'applicatif d'acquisition de photo ou de vidéo est en fonctionnement.

Une "information discriminante" est une information caractéristique qui peut être extraite d'une image (*"image feature"*), classiquement par un traitement informatique de cette image.

Une information discriminante peut présenter un nombre variable de valeurs. Par exemple, une information de contour peut être égale à 1 ou 0 selon qu'un pixel appartient ou non à un contour. Une information de brillance peut prendre un grand nombre de valeurs. Le traitement de l'image permet d'extraire et de quantifier l'information discriminante.

Les méthodes « métaheuristiques » sont des méthodes d'optimisation connues. Elles sont de préférence choisies dans le groupe formé par
- les algorithmes évolutionnistes, de préférence choisie parmi: les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
- l'algorithme du kangourou,
- la méthode de Fletcher et Powell,
- la méthode du bruitage,
- la tunnelisation stochastique,
- l'escalade de collines à recommencements aléatoires,
- la méthode de l'entropie croisée, et
- les méthodes hybrides entre les méthodes métaheuristiques citées ci-dessus.

Il faut interpréter "comprenant " ou "comportant " ou "présentant " de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente un logigramme illustrant la mise en œuvre d'un procédé de prédiction selon la description,
- la figure 2 (2a à 2e) représente des exemples de représentations graphiques fournissant des prédictions obtenues par la mise en œuvre d'un procédé selon la description pour la valeur d'un paramètre de positionnement d'une dent, en fonction du temps, dont les figures 2d et 2e représentent des exemples de représentations graphiques fournissant des prédictions obtenues par la mise en œuvre d'un procédé utilisant un système selon l'invention,
- la figure 3 représente un logigramme illustrant la mise en œuvre d'un procédé permettant d'acquérir facilement des données historiques ou actuelles,
- la figure 4 représente un exemple de modèle de référence initial,
- la figure 5 (5a-5d) illustre un traitement pour déterminer les modèles de dent dans un modèle de référence,
- la figure 6 (6a-6d) illustre l'acquisition d'une image actualisée au moyen d'un écarteur, une opération de découpage de cette image, et le traitement d'une image actualisée permettant de déterminer le contour des dents,
- la figure 7 illustre schématiquement la position relative de marques de repérage 12 d'un écarteur 10 sur des images actualisées 14₁ et 14₂, selon les directions d'observation représentées en traits interrompus,
- la figure 8 représente, sur une image acquise, une carte de référence établie pour une information de reflet.

### Description détaillée

**L'étape 1)** est une étape d'acquisition de données historiques. Elle doit commencer avant l'étape 3), mais, de préférence se poursuit pendant et postérieurement à l'étape 3).

Chaque situation historique correspond à une situation dans laquelle un patient historique s'est trouvé, et pour laquelle des données historiques ont été relevées.

Le nombre de situations historiques pour lesquelles des données historiques sont acquises est de préférence supérieur à 1 000, de préférence supérieur à 10 000, de préférence supérieur à 100 000, de préférence supérieur à 500 000.

La précision de l'analyse statistique en est améliorée.

Les situations historiques peuvent concerner différentes classes d'appareils orthodontiques. Le nombre de situations historiques relatives à une même classe d'appareils orthodontiques est de préférence supérieur à 1 000, de préférence supérieur à 10 000, de préférence supérieur à 100 000, de préférence supérieur à 500 000.

Le nombre de situations historiques relatives à une même catégorie de patients est de préférence supérieur à 1 000, de préférence supérieur à 10 000, de préférence supérieur à 100 000, de préférence supérieur à 500 000.

Les situations historiques concernent de préférence plus de 100, plus de 1000, plus de 10000, plus de 50000 patients différents.

De préférence, toutes les données historiques sont enregistrées dans une base de données informatique.

Bien entendu, le patient actuel peut avoir vécu des situations historiques, et donc avoir été lui-même un « patient historique ».

**L'étape 2)** est une étape de collecte de données actuelles relatives à la situation actuelle vécue par le patient actuel et qui sont pertinentes pour l'analyse statistique de l'étape 3).

Les données actuelles permettent de préférence de déterminer la catégorie à laquelle appartient le patient actuel.

Si le patient actuel porte un appareil orthodontique actuel, les données actuelles permettent de déterminer des paramètres de cet appareil, mais aussi, de préférence, la classe de cet appareil.

A l'étape 1) et/ou à l'étape 2), l'acquisition de données historiques ou actuelles, respectivement, peut être effectuée par tout moyen. Elle peut notamment résulter de la mise en œuvre d'un procédé d'acquisition de données comportant les étapes suivantes :
a) réalisation d'un modèle de référence tridimensionnel numérique d'au moins une partie d'une arcade, de préférence d'au moins une arcade d'un patient (actuel ou historique), ou « modèle de référence initial » et, de préférence, pour chaque dent, définition, à partir du modèle de référence initial, d'un modèle de référence tridimensionnel numérique de ladite dent, ou « modèle de dent » ;
b) acquisition d'au moins une image bidimensionnelle des arcades du patient, dite « image actualisée », dans des conditions d'acquisition réelles ;
c) analyse de chaque image actualisée et réalisation, pour chaque image actualisée, d'une carte actualisée relative à une information discriminante ;
d) optionnellement, détermination, pour chaque image actualisée, de conditions d'acquisition virtuelles grossières approximant lesdites conditions d'acquisition réelles ;
e) recherche, pour chaque image actualisée, au moyen de la carte actualisée, par déformation du modèle de référence initial, d'un modèle de référence actualisé correspondant au positionnement des dents lors de l'acquisition de l'image actualisée, la recherche étant de préférence effectuée au moyen d'une méthode métaheuristique, de préférence évolutionniste, de préférence par recuit simulé, et
f) collecte de données relatives au modèle de référence actualisé et de préférence, si le patient porte un appareil orthodontique, relatives audit appareil orthodontique.

Selon que ce procédé est mis en œuvre à l'étape 1) ou à l'étape 2), ces données collectées seront des données historiques ou actuelles, respectivement.

La fabrication du modèle de référence actualisé est avantageusement possible sans précaution particulière, notamment parce que le positionnement réel des dents est mesuré avec un modèle de référence actualisé qui résulte d'une déformation du modèle de référence initial afin qu'il corresponde aux observations fournies par les images actualisées, c'est-à-dire afin que les images actualisées soient des vues du modèle de référence initial déformé.

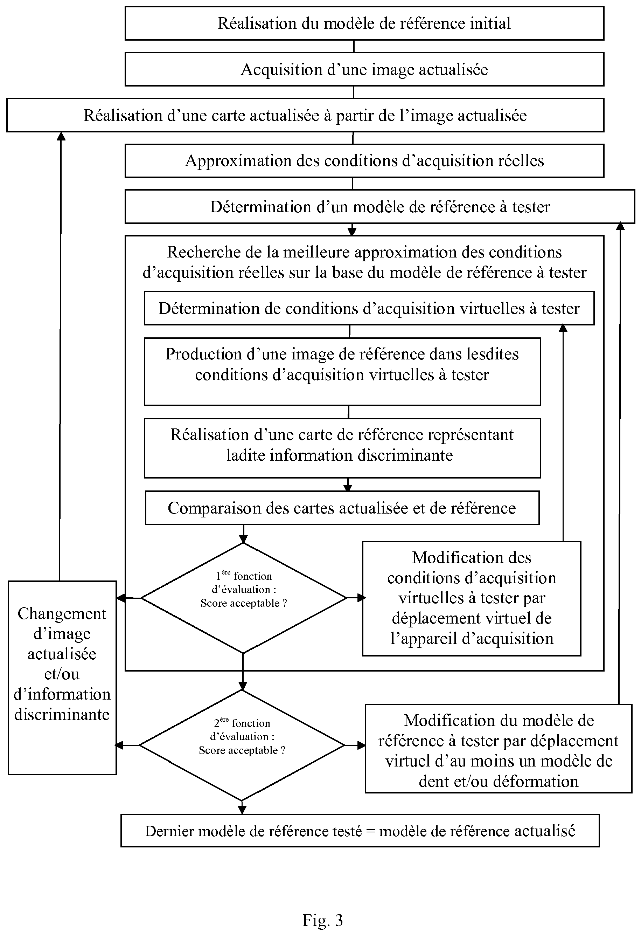

Un tel procédé d'acquisition de données, illustré sur la figure 3, permet donc, à partir d'une ou plusieurs simples images des dents, prises sans pré-positionnement précis des dents par rapport à l'appareil d'acquisition d'images, par exemple à partir d'une photographie prise par le patient, d'évaluer avec précision la position des dents au moment de l'étape b). Cette évaluation peut en outre être effectuée à distance, à partir de simples photographies prises par un téléphone mobile, sans que le patient ait à se déplacer, chez l'orthodontiste en particulier.

Si le patient porte un appareil orthodontique, cette évaluation permet ainsi avantageusement d'acquérir de nombreuses données permettant d'établir des corrélations entre des paramètres de l'appareil orthodontique, son comportement et des configurations des dents.

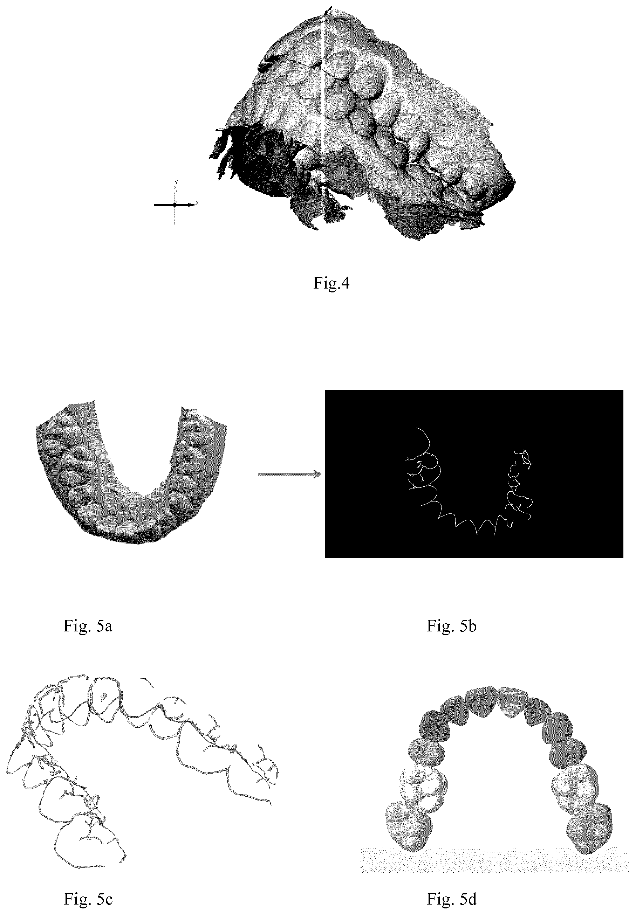

**A l'étape a),** un modèle de référence initial des arcades, ou d'une partie des arcades du patient est créé avec un scanner 3D. Un tel modèle, dit « 3D », illustré sur la figure 4, peut être observé selon un angle quelconque.

Le modèle de référence initial peut être préparé à partir de mesures effectuées sur les dents du patient ou sur un modèle physique de ses dents, par exemple un modèle en plâtre.

Dans le modèle de référence initial, une partie qui correspond à une dent, ou « modèle de dent », est délimitée par un bord gingival qui peut être décomposé en un bord gingival intérieur (du côté de l'intérieur de la bouche par rapport à la dent), un bord gingival extérieur (orienté vers l'extérieur de la bouche par rapport à la dent) et deux bords gingivaux latéraux.

**A l'étape b),** on prend une image actualisée d'une partie d'une arcade, d'une arcade ou des arcades au moyen d'un appareil d'acquisition d'images, de préférence d'un téléphone mobile.

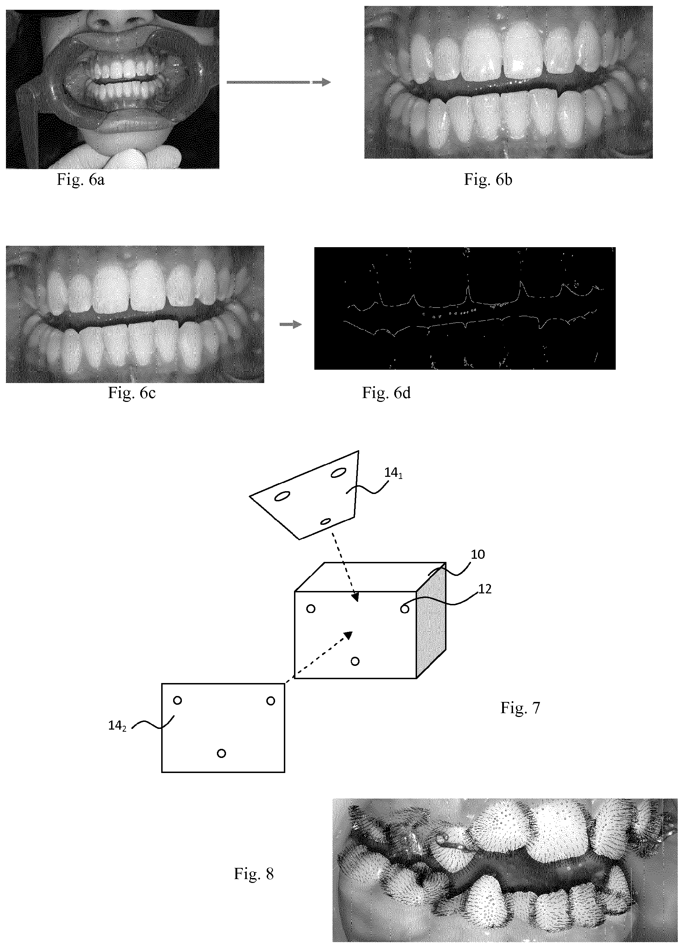

De préférence, on utilise un écarteur dentaire lors de l'étape b), comme représenté sur la figure 6a. L'écarteur comporte classiquement un support muni d'un rebord s'étendant autour d'une ouverture et agencé de manière que les lèvres du patient puissent y reposer en laissant les dents du patient apparaître à travers ladite ouverture.

**A l'étape c),** chaque image actualisée est analysée de manière à réaliser, pour chaque image actualisée, une carte actualisée relative à au moins une information discriminante.

Une carte actualisée représente une information discriminante dans le référentiel de l'image actualisée. Par exemple, la figure 5b est une carte actualisée relative au contour des dents obtenue à partir de l'image actualisée de la figure 5a.

L'information discriminante est de préférence choisie dans le groupe constitué par une information de contour, une information de couleur, une information de densité, une information de distance, une information de brillance, une information de saturation, une information sur les reflets et des combinaisons de ces informations.

L'homme de l'art sait comment traiter une image actualisée pour faire apparaître l'information discriminante.

**A l'étape optionnelle d),** on détermine, de manière grossière, les conditions d'acquisition réelles lors de l'étape b). Autrement dit, on détermine au moins la position relative de l'appareil d'acquisition d'images au moment où il a pris l'image actualisée (position de l'appareil d'acquisition dans l'espace et orientation de cet appareil). L'étape d) permet avantageusement de limiter le nombre de tests sur des conditions d'acquisition virtuelles lors de l'étape e), et permet donc d'accélérer considérablement l'étape e).

On utilise de préférence une ou plusieurs règles heuristiques. Par exemple, de préférence, on exclut des conditions d'acquisition virtuelles susceptibles d'être testées à l'étape e), les conditions qui correspondent à une position de l'appareil d'acquisition d'images derrière les dents ou à une distance des dents supérieure à 1 m.

Dans un mode de réalisation préféré, comme illustré sur la figure 7, on utilise des marques de repérage représentées sur l'image actualisée, et en particulier des marques de repérage 12 de l'écarteur, pour déterminer une région de l'espace sensiblement conique délimitant des conditions d'acquisition virtuelles susceptibles d'être testées à l'étape e), ou "cône de test".

Précisément, on dispose de préférence au moins trois marques de repérage 12 non alignées sur l'écarteur 10, et on mesure précisément leurs positions relatives sur l'écarteur.

Les marques de repérage sont ensuite repérées sur l'image actualisée, comme décrit précédemment. De simples calculs trigonométriques permettent de déterminer approximativement la direction selon laquelle l'image actualisée a été prise.

L'étape d) ne permet qu'une évaluation grossière des conditions d'acquisition réelles. L'étape d) permet cependant de déterminer un ensemble restreint de conditions d'acquisition virtuelles susceptibles de correspondre aux conditions d'acquisition réelles, et, dans cet ensemble, des conditions d'acquisition virtuelles constituant le meilleur point de départ pour l'étape e) décrite ci-après.

L'objectif de **l'étape e)** est de modifier le modèle de référence initial jusqu'à obtenir un modèle de référence actualisé qui corresponde à l'image actualisée. Idéalement, le modèle de référence actualisé est donc un modèle de référence tridimensionnel numérique à partir duquel l'image actualisée aurait pu être prise si ce modèle avait été réel.

On teste donc une succession de modèles de référence « à tester », le choix d'un modèle de référence à tester étant de préférence dépendant du niveau de correspondance des modèles de référence « à tester » précédemment testés avec l'image actualisée. Ce choix est de préférence effectué en suivant un procédé d'optimisation connu, en particulier choisi parmi les procédés d'optimisation métaheuristiques, de préférence évolutionnistes, en particulier dans les procédés de recuit simulé.

De préférence, l'étape e) comporte
- une première opération d'optimisation permettant de rechercher des conditions d'acquisition virtuelles correspondant au mieux aux conditions d'acquisition réelles dans un modèle de référence à tester déterminé à partir du modèle de référence initial, et
- une deuxième opération d'optimisation permettant de rechercher, en testant une pluralité de dits modèles de référence à tester, le modèle de référence correspondant au mieux au positionnement des dents du patient lors de l'acquisition de l'image actualisée à l'étape b).

De préférence, une première opération d'optimisation est effectuée pour chaque test d'un modèle de référence à tester lors de la deuxième opération d'optimisation.

De préférence, la première opération d'optimisation et/ou la deuxième opération d'optimisation, de préférence la première opération d'optimisation et la deuxième opération d'optimisation mettent en œuvre une méthode métaheuristique, de préférence évolutionniste, de préférence un recuit simulé.

De préférence, l'étape e) comporte les étapes suivantes :
e1) définition d'un modèle de référence à tester comme étant le modèle de référence initial puis,
e2) suivant les étapes suivantes, test de conditions d'acquisition virtuelles avec le modèle de référence à tester afin d'approximer finement lesdites conditions d'acquisition réelles ;
   e21) détermination de conditions d'acquisition virtuelles à tester;
   e22)réalisation d'une image de référence bidimensionnelle du modèle de référence à tester dans lesdites conditions d'acquisition virtuelles à tester ;
   e23)traitement de l'image de référence pour réaliser au moins une carte de référence représentant, au moins partiellement, ladite information discriminante ;
   e24)comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e21) ;
   e25) si ladite valeur pour la première fonction d'évaluation correspond à une décision de poursuivre ladite recherche, modification des conditions d'acquisition virtuelles à tester, puis reprise à l'étape e22) ;
e3) détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e2), ladite valeur pour la deuxième fonction d'évaluation correspondant à une décision de poursuivre ou de stopper la recherche d'un modèle de référence approximant le positionnement des dents lors de l'acquisition de l'image actualisée avec plus d'exactitude que ledit modèle de référence à tester utilisé à la dernière occurrence de l'étape e2), et si ladite valeur pour la deuxième fonction d'évaluation correspond à une décision de poursuivre ladite recherche, modification du modèle de référence à tester par déplacement d'un ou plusieurs modèles de dents, puis reprise à l'étape e2).

**A l'étape e1),** on détermine que le modèle de référence à tester est le modèle de référence initial lors de la première exécution de l'étape e2).

**A l'étape e2),** on commence par déterminer des conditions d'acquisition virtuelles à tester, c'est-à-dire une position et une orientation virtuelles susceptibles de correspondre à la position et l'orientation réelles de l'appareil d'acquisition lors de la capture de l'image actualisée, mais aussi, de préférence, une calibration virtuelle susceptible de correspondre à la calibration réelle de l'appareil d'acquisition lors de la capture de l'image actualisée.

On configure ensuite virtuellement l'appareil d'acquisition d'images dans les conditions d'acquisition virtuelles à tester afin d'acquérir une image de référence du modèle de référence à tester dans ces conditions d'acquisition virtuelles à tester. L'image de référence correspond donc à l'image qu'aurait prise l'appareil d'acquisition d'images s'il avait été placé, par rapport au modèle de référence à tester, et optionnellement calibré, dans les conditions d'acquisition virtuelles à tester (étape e22)).

Si l'image actualisée a été prise alors que la position des dents était exactement celle dans le modèle de référence à tester, et si les conditions d'acquisition virtuelles sont exactement les conditions d'acquisition réelles, l'image de référence est donc exactement superposable à l'image actualisée. Les différences entre l'image actualisée et l'image de référence résultent d'erreurs dans l'évaluation des conditions d'acquisition virtuelles (si elles ne correspondent pas exactement aux conditions d'acquisition réelles) et de déplacements des dents entre l'étape b) et le modèle de référence à tester.

Pour comparer les images actualisée et de référence, on compare l'information discriminante sur ces deux images. Plus précisément, on réalise, à partir de l'image de référence, une carte de référence représentant l'information discriminante (étape e23)).

Les cartes actualisée et de référence, portant toutes les deux sur la même information discriminante, sont ensuite comparées et on évalue la différence entre ces deux cartes au moyen d'un score. Par exemple, si l'information discriminante est le contour des dents, on peut comparer la distance moyenne entre les points du contour des dents qui apparait sur l'image de référence et les points du contour correspondant qui apparaît sur l'image actualisée, le score étant d'autant plus élevé que cette distance est faible.

De préférence, les conditions d'acquisition virtuelles comprennent les paramètres de calibration de l'appareil d'acquisition. Le score est d'autant plus élevé que les valeurs des paramètres de calibration testées sont proches des valeurs des paramètres de calibration de l'appareil d'acquisition utilisé à l'étape b). Par exemple, si l'ouverture de diaphragme testée est éloignée de celle de l'appareil d'acquisition utilisé à l'étape b), l'image de référence présente des régions floues et des régions nettes qui ne correspondent pas aux régions floues et aux régions nettes de l'image actualisée. Si l'information discriminante est le contour des dents, les cartes actualisée et de référence ne représenteront donc pas les mêmes contours et le score sera faible.

Le score peut être par exemple un coefficient de corrélation.

Le score est ensuite évalué au moyen d'une première fonction d'évaluation. La première fonction d'évaluation permet de décider si le cyclage sur l'étape e2) doit être poursuivi ou stoppé. La première fonction d'évaluation peut par exemple être égale à 0 si le cyclage doit être stoppé ou être égale à 1 si le cyclage doit se poursuivre.

La valeur de la première fonction d'évaluation peut dépendre du score atteint. Par exemple, il peut être décidé de poursuivre le cyclage sur l'étape e2) si le score ne dépasse pas un premier seuil. Par exemple, si une correspondance exacte entre les images actualisée et de référence conduit à un score de 100%, le premier seuil peut être, par exemple, de 95%. Bien entendu, plus le premier seuil sera élevé, meilleure sera la précision de l'évaluation des conditions d'acquisition virtuelles si le score parvient à dépasser ce premier seuil.

La valeur de la première fonction d'évaluation peut également dépendre de scores obtenus avec des conditions d'acquisition virtuelles testées précédemment.

La valeur de la première fonction d'évaluation peut également dépendre de paramètres aléatoires et/ou du nombre de cycles de l'étape e2) déjà effectués.

En particulier, il est possible qu'en dépit de la répétition des cycles, on ne parvienne pas à trouver des conditions d'acquisition virtuelles qui soient suffisamment proches des conditions d'acquisition réelles pour que le score atteigne ledit premier seuil. La première fonction d'évaluation peut alors conduire à la décision de quitter le cyclage bien que le meilleur score obtenu n'ait pas atteint ledit premier seuil. Cette décision peut résulter, par exemple, d'un nombre de cycles supérieur à un nombre maximal prédéterminé.

Un paramètre aléatoire dans la première fonction d'évaluation peut également autoriser la poursuite de tests de nouvelles conditions d'acquisition virtuelles, bien que le score apparaisse satisfaisant.

Les fonctions d'évaluation classiquement utilisées dans les procédés d'optimisation métaheuristiques, de préférence évolutionnistes, en particulier dans les procédés de recuit simulé, peuvent être utilisées pour la deuxième fonction d'évaluation.

Si la valeur de la première fonction d'évaluation indique qu'il est décidé de poursuivre le cyclage sur l'étape e2), on modifie les conditions d'acquisition virtuelles testées (étape e25)) et on recommence un cycle (étape e2)) consistant à réaliser une image de référence et une carte de référence, puis à comparer cette carte de référence avec la carte actualisée pour déterminer un score.

La modification des conditions d'acquisition virtuelles correspond à un déplacement virtuel dans l'espace et/ou à une modification de l'orientation et/ou, de préférence, à une modification de la calibration de l'appareil d'acquisition. Cette modification peut être aléatoire, à condition cependant que les nouvelles conditions d'acquisition virtuelles à tester appartiennent toujours à l'ensemble déterminé à l'étape d). La modification est de préférence guidée par des règles heuristiques, par exemple en favorisant les modifications qui, d'après une analyse des précédents scores obtenus, apparaissent les plus favorables pour augmenter le score.

Le cyclage sur e2) est poursuivi jusqu'à ce que la valeur de la première fonction d'évaluation indique qu'il est décidé de sortir de ce cyclage et de poursuivre à l'étape e3), par exemple si le score atteint ou dépasse ledit premier seuil.

L'optimisation des conditions d'acquisition virtuelles à l'étape e2) est de préférence effectuée en utilisant une méthode métaheuristique, de préférence évolutionniste, de préférence un algorithme de recuit simulé. Un tel algorithme est bien connu pour l'optimisation non linéaire.

Si on a quitté le cyclage sur l'étape e2), sans qu'un score satisfaisant ait pu être obtenu, par exemple sans que le score ait pu atteindre ledit premier seuil, le procédé peut être arrêté (situation d'échec) ou repris à l'étape c) avec une nouvelle information discriminante et/ou avec une nouvelle image actualisée. Le procédé peut être également poursuivi avec les conditions d'acquisition virtuelles correspondant au meilleur score atteint. Un avertissement peut être émis afin d'informer l'utilisateur de l'erreur sur le résultat.

Si on a quitté le cyclage sur l'étape e2) alors qu'un score satisfaisant a pu être obtenu, par exemple parce que le score a atteint, voire dépassé ledit premier seuil, les conditions d'acquisition virtuelles correspondent sensiblement aux conditions d'acquisition réelles.

De préférence, les conditions d'acquisition virtuelles comprennent les paramètres de calibration de l'appareil d'acquisition. Le procédé conduit permet ainsi d'évaluer les valeurs de ces paramètres sans qu'il soit nécessaire de connaître la nature de l'appareil d'acquisition ou son réglage. L'étape b) peut donc être réalisée sans précaution particulière, par exemple par le patient lui-même au moyen de son téléphone portable.

En outre, la recherche de la calibration réelle est effectuée en comparant une image actualisée avec des vues d'un modèle de référence initial dans des conditions d'acquisition virtuelles que l'on teste. Avantageusement, elle ne nécessite pas que l'image actualisée fasse apparaître une jauge étalon de calibration, c'est-à-dire une jauge dont on connaît précisément les caractéristiques permettant déterminer la calibration de l'appareil d'acquisition.

Les images actualisées ne servent pas à créer un modèle tridimensionnel actualisé totalement nouveau, mais seulement à modifier le modèle de référence initial, très précis. Un modèle tridimensionnel actualisé totalement nouveau créé à partir de simples photographies prises sans précautions particulières serait en particulier trop imprécis pour qu'une comparaison avec le modèle de référence initial puisse conduire à des conclusions sur le déplacement des dents.

Des différences peuvent subsister entre les conditions d'acquisition virtuelles déterminées et les conditions d'acquisition réelles, en particulier si des dents se sont déplacées entre les étapes a) et b). La corrélation entre les images actualisée et de référence peut alors être encore améliorée en reprenant l'étape e2), le modèle de référence à tester étant alors modifié par déplacement d'un ou plusieurs modèles de dents (étape e3)).

La recherche du modèle de référence approximant au mieux le positionnement des dents lors de l'acquisition de l'image actualisée peut être effectuée comme la recherche des conditions d'acquisition virtuelles approximant au mieux les conditions d'acquisition réelles (étape e2)).

En particulier, le score est évalué au moyen d'une deuxième fonction d'évaluation. La deuxième fonction d'évaluation permet de décider si le cyclage sur les étapes e2) et e3) doit être poursuivi ou stoppé. La deuxième fonction d'évaluation peut par exemple être égale à 0 si le cyclage doit être stoppé ou être égale à 1 si le cyclage doit se poursuivre.

La valeur de la deuxième fonction d'évaluation dépend de préférence du meilleur score obtenu avec le modèle de référence à tester, c'est-à-dire des différences entre les cartes actualisée et de référence, dans les conditions d'acquisition virtuelles approximant au mieux lesdites conditions d'acquisition réelles.

La valeur de la deuxième fonction d'évaluation peut également dépendre du meilleur score obtenu avec un ou plusieurs modèles de référence testés précédemment.

Par exemple, il peut être décidé de poursuivre le cyclage si le score ne dépasse pas un deuxième seuil minimal. La valeur de la deuxième fonction d'évaluation peut également dépendre de paramètres aléatoires et/ou du nombre de cycles des étapes e2) et e3) déjà effectués.

Les fonctions d'évaluation classiquement utilisées dans les procédés d'optimisation métaheuristiques, de préférence évolutionnistes, en particulier dans les procédés de recuit simulé, peuvent être utilisées pour la deuxième fonction d'évaluation.

Si la valeur de la deuxième fonction d'évaluation indique qu'il est décidé de poursuivre le cyclage sur les étapes e2) et e3), on modifie le modèle de référence à tester et on recommence un cycle (étapes e2) et e3)) avec le nouveau modèle de référence à tester.

La modification du modèle de référence à tester correspond à un déplacement d'un ou plusieurs modèles de dents. Cette modification peut être aléatoire. La modification est de préférence guidée par des règles heuristiques, par exemple en favorisant les modifications qui, d'après une analyse des précédents scores obtenus, apparaissent les plus favorables pour augmenter le score.

De préférence, on recherche le déplacement d'un modèle de dent qui a le plus fort impact sur le score, on modifie le modèle de référence à tester en déplaçant ce modèle de dent, puis on poursuit le cyclage sur les étapes e2) et e3) de manière à optimiser le score. On peut ensuite rechercher, parmi les autres modèles de dent, celui qui a le plus fort impact sur l'amélioration du score, et à nouveau rechercher le déplacement optimal de cet autre modèle de dent sur le score. On peut poursuivre ainsi avec chaque modèle de dent.

Ensuite, il est possible de reprendre un cycle sur l'ensemble des modèles de dent et de poursuivre ainsi jusqu'à l'obtention d'un score supérieur au deuxième seuil. Bien entendu, d'autres stratégies peuvent être utilisées pour déplacer un ou plusieurs modèles de dent dans le modèle de référence à tester et rechercher le score maximal.

Le cyclage sur les étapes e2) et e3) est poursuivi jusqu'à ce que la valeur de la deuxième fonction d'évaluation indique qu'il est décidé de sortir de ce cyclage et de poursuivre à l'étape f), par exemple si le score atteint ou dépasse ledit deuxième seuil.

La recherche d'un modèle de référence avec un cyclage sur les étapes e2) et e3) pour rechercher les positions des modèles de dent qui optimisent le score est de préférence effectuée en utilisant une méthode métaheuristique, de préférence évolutionniste, de préférence un algorithme de recuit simulé. Un tel algorithme est bien connu pour l'optimisation non linéaire.

Si on a quitté le cyclage sur les étapes e2) et e3) sans qu'un score satisfaisant ait pu être obtenu, par exemple sans que le score ait pu atteindre ledit deuxième seuil, le procédé peut être arrêté (situation d'échec) ou repris à l'étape c) avec une nouvelle information discriminante et/ou avec une nouvelle image actualisée.

S'il est décidé de relancer le procédé à l'étape c) à partir d'une autre information discriminante et/ou d'une autre image actualisée parce que le premier seuil ou le deuxième seuil n'a pas été atteint, le choix de la nouvelle information discriminante et/ou de la nouvelle image actualisée peut dépendre des scores obtenus précédemment, afin de favoriser l'information discriminante et/ou l'image actualisée qui, au regard de ces scores, apparaissent les plus prometteuses.

Une nouvelle information discriminante, obtenue par exemple par combinaison d'autres informations discriminantes déjà testées, peut être utilisée. Le cas échéant, il peut être également demandé d'acquérir une ou plusieurs nouvelles images actualisées. De préférence, on fournit des indications permettant de guider le positionnement de l'appareil d'acquisition pour la capture de cette nouvelle image actualisée. Par exemple, on peut indiquer au patient qu'il devrait prendre une photo de la partie droite de son arcade inférieure.

Si on a quitté le cyclage sur les étapes e2) et e3) sans qu'un score satisfaisant ait pu être obtenu, un avertissement peut être émis afin d'informer l'utilisateur de l'erreur sur le résultat.

Si on a quitté le cyclage sur les étapes e2) et e3) alors qu'un score satisfaisant a pu être obtenu, par exemple parce que le score a atteint, voire dépassé ledit deuxième seuil, les conditions d'acquisition virtuelles correspondent sensiblement aux conditions d'acquisition réelles et les modèles de dents dans le modèle de référence obtenu (dit « modèle de référence actualisé ») sont sensiblement dans la position des dents du patient au moment de l'étape b).

Le cyclage sur les étapes e2) et e3) permet avantageusement d'améliorer l'évaluation des paramètres de calibration de l'appareil d'acquisition à l'étape b).

A l'issue de l'étape e), le modèle de référence actualisé correspond sensiblement à l'image actualisée. Il est alors possible de faire des mesures précises sur le positionnement des dents et/ou sur la forme de l'appareil orthodontique afin d'acquérir des données historiques ou actuelles.

Les étapes a) à e) peuvent comporter une ou plusieurs caractéristiques des étapes correspondantes du procédé décrit dans PCT/EP2015/074896, incorporé par référence, publié sous le numéro WO 2016 066651 (le « modèle de référence final » mentionné dans l'étape e) de WO 2016 066651 correspondant au présent « modèle de référence actualisé »).

**A l'étape 3),** toutes les méthodes d'analyse statistique peuvent être utilisées.

Avantageusement, l'analyse statistique permet d'établir des corrélations entre des données historiques, et en particulier entre des valeurs de paramètres de l'appareil orthodontique éventuellement porté et son comportement, sans qu'il soit nécessaire d'établir analytiquement chacune des corrélations.

L'analyse statistique est de préférence mise en œuvre par un programme d'ordinateur.

L'analyse statistique comporte une analyse des données historiques afin de prédire la valeur d'un ou plusieurs paramètres de contexte à un instant futur en fonction d'un ensemble de contraintes imposées pour cette prédiction, et notamment des données actuelles.

L'analyse statistique permet de prédire à un instant futur, de préférence à tout instant futur, la valeur d'au moins un paramètre de positionnement de dents du patient actuel, par exemple la position théorique d'un point quelconque d'une dent.

Plus précisément, l'analyse statistique permet de construire, à partir des données historiques, un modèle prédictif permettant de prévoir comment la valeur de certains paramètres va évoluer en fonction de « données d'entrée ».

De préférence, les données historiques comprennent les valeurs de paramètres de contexte relatives à des situations dentaires historiques vécues par le patient actuel. De préférence, l'analyse statistique affecte un poids à ces valeurs qui est plus élevé que celui de valeurs de ces paramètres de contexte relatives à des situations dentaires historiques vécues par d'autres patients.

Ainsi, par exemple, si le patient actuel suit un traitement orthodontique, l'analyse des données historiques d'autres traitements orthodontiques, par exemple de 1000 autres traitement orthodontiques similaires, peut conduire à estimer que le déplacement du barycentre d'une dent du patient selon l'axe Ox) dans le mois qui suit l'instant actuel devrait être de 60 µm. Si l'analyse des données historiques du seul patient actuel conduit à estimer ce déplacement à 50 µm, l'analyse statistique pourrait par exemple estimer ce déplacement à 55 µm. Dans cet exemple, le poids des données historiques du patient est donc 1000 fois supérieur à celui des données historiques des autres patients.

Les données d'entrée sont des valeurs qui figent les valeurs de certains paramètres.

Les valeurs de certains paramètres ne peuvent être que des données d'entrée. Ces paramètres constituent ainsi des contraintes non ajustables.

Par exemple, la prédiction s'effectue nécessairement à partir de la position des dents du patient actuel à l'instant actuel. Les valeurs des paramètres de positionnement de dents du patient actuel à l'instant actuel sont donc des données d'entrée.

De même, si le patient actuel suit un traitement orthodontique, la prédiction s'effectue à partir d'une situation dentaire actuelle dans laquelle un appareil orthodontique est porté sur les dents dans leur position actuelle. La valeur de certains paramètres de l'appareil orthodontique à l'instant actuel, par exemple la position des points d'attache, peut donc également être imposée. La classe de l'appareil orthodontique actuel ou ses paramètres intrinsèques sont d'autres exemples de contraintes non ajustables.

Les paramètres qui ne sont pas des contraintes non ajustables peuvent être figées ou n'être autoriser à varier que dans des plages figées. Ils constituent des contraintes ajustables, notamment par l'orthodontiste et/ou le patient.

Par exemple, la durée totale d'un traitement orthodontique peut être figée à 6 mois. Un coefficient de douleur mesurant la douleur maximale pendant un traitement orthodontique peut être également figé.

Dans un mode de réalisation préféré, l'instant futur est un instant futur « objectif», c'est-à-dire correspondant à un instant prédéterminé du traitement, en particulier correspondant à un set-up intermédiaire ou final, et une plage pour les valeurs possibles d'un paramètre de positionnement des dents est imposée à cet instant futur. Ce paramètre de positionnement est alors une contrainte ajustable.

Un paramètre ne constitue une contrainte ajustable que si, pour l'analyse statistique, il est décidé de limiter le domaine de ses valeurs possibles. Sinon, il s'agit d'un paramètre libre.

Les paramètres qui ne sont pas des contraintes sont donc libres de varier. Si les domaines des valeurs possibles pour les contraintes ajustables sont suffisamment larges et si les contraintes ajustables ne sont pas trop nombreuses, l'analyse statistique permettra de prédire au moins une situation dentaire future respectant les contraintes. Sinon, de simples essais permettront, en relâchant certaines contraintes, d'obtenir au moins une prédiction.

De nombreux paramètres peuvent être des contraintes ajustables. L'analyse statistique permet donc souvent de déterminer plusieurs « traitements orthodontiques potentiels » pour améliorer la situation dentaire du patient actuel. De simples essais permettent de limiter les plages de valeurs possibles pour les contraintes ajustables afin de limiter le nombre de traitements orthodontiques potentiels.

De préférence, si le patient actuel suit un traitement orthodontique, l'analyse statistique n'utilise que des données historiques de situations dentaires relatives à des appareils orthodontiques de la même classe que l'appareil orthodontique actuel. La précision de la prédiction en est améliorée.

De préférence, l'analyse statistique n'utilise que des données historiques de situations dentaires relatives à des patients historiques de la même catégorie que le patient actuel. La précision de la prédiction en est améliorée.

Un instant futur peut être notamment postérieur à l'instant actuel de plus d'une semaine, plus de 2 semaines, plus de 3 semaines, plus de 5 semaines, plus de 8 semaines, plus de 10 semaines. La prédiction est d'autant plus précise que l'instant futur considéré est proche de l'instant actuel.

De préférence, on détermine une situation dentaire future pour un instant futur correspondant à un set-up intermédiaire ou final.

Lorsque l'analyse statistique permet de déterminer plusieurs situations dentaires futures pour un même instant futur, elle sélectionne de préférence au moins une, de préférence deux situations dentaires futures correspondant à des situations dentaires extrêmes, c'est-à-dire à des situations correspondant à des valeurs limites acceptables, à cet instant futur, pour une contrainte ajustable. La contrainte ajustable peut être en particulier un paramètre de positionnement d'une dent pour lequel l'orthodontiste et/ou le patient ont fixé une plage de valeurs possibles.

De préférence, on détermine au moins une situation dentaire future pour plusieurs instants futurs différents.

Lorsqu'on détermine au moins une situation dentaire future pour plusieurs instants futurs différents, les différents instants futurs peuvent être notamment séparés de plus de 3 jours, plus d'une semaine ou plus de 2 semaines, et/ou moins de 2 mois, ou moins d'un mois.

De préférence, au moins un des instants futurs est un instant futur "objectif".

L'analyse statistique permet ainsi de prédire un traitement orthodontique potentiel jusqu'à l'instant futur objectif. En fixant les contraintes ajustables, il est possible de prédire plusieurs traitements orthodontiques potentiels et donc de mesurer l'impact d'un choix sur les contraintes ajustables.

Les résultats de l'analyse statistique peuvent être présentés sous une forme quelconque.

De préférence, on présente l'écart entre une situation dentaire future pour un instant futur et une prédiction de la situation dentaire anticipée, pour ledit instant futur, à un instant antérieur à l'instant actuel, par exemple en début de traitement. Cette présentation de l'écart est en particulier utile lorsque l'instant futur correspond à un set-up intermédiaire ou final.

De préférence, on présente l'écart entre un traitement orthodontique potentiel et une prédiction correspondante du traitement orthodontique anticipée à un instant antérieur à l'instant actuel, par exemple en début de traitement. Cette présentation de l'écart permet de visualiser si le traitement s'est déroulé et va se dérouler conformément à la prédiction initiale.

De préférence, on présente plusieurs situations dentaires futures, à des instants futurs différents, sur une même représentation graphique.

De préférence, on établit, à partir du résultat de l'analyse statistique, une représentation graphique présentant une prédiction de l'évolution temporelle d'au moins un paramètre de contexte.

L'évolution temporelle peut être notamment celle de :
- un paramètre de l'appareil orthodontique actuel ; et/ou
- un paramètre de positionnement des dents du patient actuel, par exemple l'évolution temporelle du déplacement d'un point d'une dent ; et/ou
- une probabilité que la situation dentaire future, à un instant futur considéré se réalise ; et/ou
- un écart avec une situation dentaire constituant un objectif à l'instant futur considéré ; et/ou
- un coût prévisible pour que la situation dentaire future à un instant futur considéré se réalise ; et/ou
- un coefficient de douleur pour que la situation dentaire à un instant futur considéré se réalise.

L'échelle du temps est de préférence linéaire. Une représentation graphique permet par exemple de percevoir immédiatement la dynamique de l'action de l'appareil orthodontique. En particulier, le constat que la courbe d'un paramètre de positionnement croît ou décroît moins vite à mesure que le temps s'écoule, peut être interprété comme signifiant que l'appareil orthodontique perd de son efficacité.

Une telle courbe est particulièrement simple à comprendre, ce qui rend son utilisation possible par le patient actuel lui-même.

Dans un mode de réalisation préféré, la représentation graphique comporte des indications sur le caractère satisfaisant ou insatisfaisant de ladite évolution. Par exemple, la courbe peut changer de couleur si la pente est considérée comme anormale. Il est ainsi possible de déterminer, par exemple, le moment à partir duquel tout retard dans l'adaptation du traitement sera préjudiciable.

De préférence, la représentation graphique peut être affichée sur un téléphone portable. En particulier, elle est visualisable par le patient. Avantageusement, le patient peut ainsi décider de lui-même, au moment le plus opportun, de prendre des mesures pour modifier ou changer son appareil orthodontique, ou de prendre rendez-vous chez l'orthodontiste.

Dans un mode de réalisation, le nombre de paramètres dont l'évolution est représentée graphiquement est inférieur à 10, de préférence inférieur à 5, de préférence inférieur à 4, de préférence inférieur à 3, de préférence inférieur à 2. Dans un mode de réalisation, le nombre de points de dent pour lesquels l'évolution d'un ou plusieurs paramètres est représentée graphiquement est inférieur à 10, de préférence inférieur à 5, de préférence inférieur à 4, de préférence inférieur à 3, de préférence inférieur à 2. La prise de décision en est facilitée.

Le rapport peut notamment préciser comment modifier la tension de l'arc de l'appareil orthodontique actuel ou comment fabriquer une nouvelle gouttière.

De préférence, on détermine plusieurs traitements orthodontiques potentiels permettant, à partir de la situation dentaire actuelle, d'atteindre un positionnement des dents souhaité, puis on présente lesdits traitements potentiels au patient et/ou à l'orthodontiste de manière qu'il(s) choisisse(nt) un desdits traitements orthodontiques potentiels.

De préférence, la modification des contraintes ajustables est renouvelée dans le cadre d'une optimisation. A chaque cycle d'optimisation, une ou plusieurs desdites contraintes ajustables sont modifiées, puis
- on évalue la situation dentaire future obtenue jusqu'à trouver une situation dentaire optimale au regard d'un critère d'optimisation, suivant au moins une règle d'optimisation, et/ou
- de préférence, on évalue le traitement orthodontique potentiel obtenu jusqu'à trouver un traitement orthodontique optimal au regard d'un critère d'optimisation, suivant au moins une règle d'optimisation,

Bien entendu, toutes les méthodes d'optimisation connues peuvent être mises en œuvre.

Plus précisément, pour effectuer une optimisation, certaines contraintes ajustables sont classiquement figées, d'autres sont variables dans une plage qui est figée.

Pour l'optimisation d'une situation dentaire future, le critère d'optimisation peut être par exemple un coefficient de douleur ou un coût associés à cette situation.

L'objectif de l'optimisation peut être, par exemple, de minimiser le coefficient de douleur à un instant futur. On effectue alors une première analyse statistique avec un premier coefficient de douleur et on examine si le résultat est acceptable. Si le résultat est acceptable, on réduit le coefficient de douleur et on examine si le nouveau résultat est acceptable. On modifie ainsi des contraintes jusqu'à déterminer le coefficient de douleur le plus faible permettant d'atteindre un résultat acceptable.

Pour l'optimisation d'un traitement orthodontique, le critère d'optimisation peut être par exemple un écart avec une valeur souhaitée pour un paramètre de positionnement à un instant futur objectif, par exemple à la fin du traitement. Le critère d'optimisation peut être aussi un coefficient de douleur moyen évaluant, en moyenne, la douleur jusqu'à la fin du traitement, ou un coût total du traitement.

A chaque cycle d'optimisation, il est alors nécessaire de prédire un traitement orthodontique potentiel , de mesurer le critère d'optimisation pour ce traitement orthodontique potentiel, par exemple en faisant la moyenne des coefficients de douleur associés à chaque situation dentaire future du traitement orthodontique potentiel, puis de modifier une ou plusieurs contraintes ajustables en respectant les plages dans lesquelles ces contraintes variables peuvent varier.

En comparant les valeurs du critère d'optimisation obtenues pour les différents cycles, on peut ainsi rechercher le traitement orthodontique potentiel optimal.

La règle d'optimisation peut être par exemple de minimiser ou de maximiser la valeur du critère d'optimisation.

L'optimisation est de préférence effectuée par un ordinateur, l'orthodontiste ou le patient précisant seulement des contraintes ajustables, par exemple une valeur maximale ou une plage acceptable pour les contraintes ajustables.

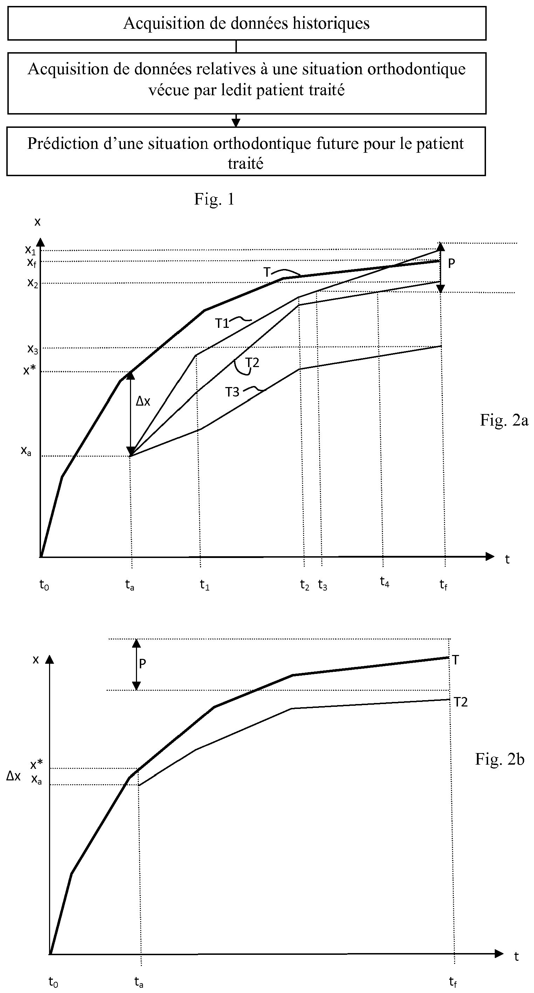

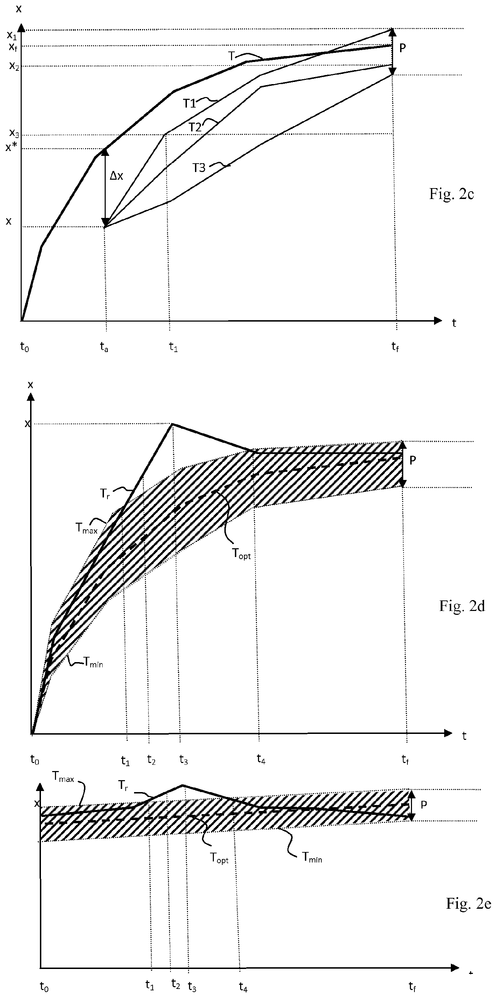

Par exemple, l'orthodontiste peut imposer un coût maximal, une durée maximale, un coefficient de douleur maximal, un nombre de rendez-vous maximal ou un nombre de gouttières maximal. Il peut également préciser, en fonction de l'objectif du traitement orthodontique, la plage de valeurs acceptables pour un ou plusieurs paramètres de contexte, et en particulier paramètres de positionnement (plage P sur la figure 2, décrite ci-après).

Par analyse statistique des données historiques, l'ordinateur et/ou l'orthodontiste et/ou le patient actuel recherchent alors une ou, de préférence plusieurs situations dentaires futures, de préférence un ou, de préférence plusieurs traitements orthodontiques potentiels permettant de répondre à ces contraintes ajustables.

Dans un mode de réalisation, si les contraintes ajustables imposées ne permettent pas de déterminer un traitement orthodontique potentiel respectant ces contraintes, l'orthodontiste les modifie, puis renouvelle l'analyse statistique et l'optimisation. Il recommence ces opérations jusqu'à avoir trouvé au moins un traitement orthodontique potentiel.

Par exemple, si le patient veut que son traitement dure moins de 3 mois, une contrainte ajustable liée à la douleur ou au coût pourra être au moins partiellement levée.

Pour explorer au mieux les différents traitements possibles, il est également possible de lever la contrainte relative à un ou plusieurs paramètres de positionnement des dents à un instant futur objectif. Par exemple, sur la figure 2a, décrite ci-après, la courbe T3 n'a pu être réalisée que parce que la contrainte selon laquelle, à l'instant futur objectif t_{f}, la valeur du paramètre de positionnement *x* doit être comprise dans la plage P a été levée. En revanche, cette contrainte n'a pas été levée dans l'exemple de la figure 2c.

L'optimisation peut être également partiellement manuelle.

**A l'étape 4),** l'orthodontiste analyse les résultats obtenus à l'étape 3) et détermine éventuellement un traitement orthodontique ou modifie un traitement orthodontique actuel en fonction de ces résultats et, de préférence, en fonction de choix du patient actuel.

La capacité de prédiction offerte par l'invention permet notamment de déterminer un traitement orthodontique en fonction de contraintes comme le coût du traitement, le nombre de rendez-vous chez l'orthodontiste, la forme ou le réglage de l'appareil orthodontique, la douleur, la durée du traitement ou la probabilité de succès.

L'orthodontiste peut considérer que la situation est acceptable et décider de ne pas mettre en œuvre de traitement orthodontique pour le patient actuel ou de ne pas modifier un traitement orthodontique suivi par le patient actuel. Alternativement, il peut décider de fabriquer un appareil orthodontique ou de modifier l'appareil orthodontique actuel éventuellement porté par le patient actuel, ou le changer.

L'orthodontiste peut en particulier modifier la tension d'un arc orthodontique de l'appareil orthodontique actuel et/ou changer un arc orthodontique de l'appareil orthodontique actuel et/ou on fabriquer une nouvelle gouttière orthodontique pour remplacer l'appareil orthodontique actuel. Dans un mode de réalisation, l'appareil orthodontique actuel est une gouttière, et on envoie au patient une deuxième gouttière fabriquée en fonction des résultats de l'analyse statistique de l'étape 3).

L'appareil orthodontique est ainsi bien adapté à la réalité du traitement.

L'orthodontiste peut également avantageusement décider de modifier le traitement initialement envisagé, notamment en modifiant les set-ups intermédiaires.

Dans le cas d'un traitement orthodontique au moyen de gouttières, le procédé permet de limiter le nombre de gouttières fabriquées. Les gouttières peuvent être en particulier fabriquées tout au long du traitement, ce qui leur permet d'être parfaitement adaptées à la situation réelle au moment où elles doivent être utilisées.

Enfin, le coût et la durée de traitement sont réduits.

### Exemples

Sur la figure 2a, on a représenté trois traitements orthodontiques potentiels T1, T2 et T3 correspondant à trois ensembles de contraintes différents. Pour chaque traitement orthodontique potentiel, une situation dentaire future a été évaluée, par analyse statistique, à des instants t₁, t₂ et t_{f}.

L'axe des abscisses fournit le temps. L'axe des ordonnées fournit la valeur d'un paramètre de contexte x, par exemple la position d'un point d'une dent selon un axe Ox).

La plage P est une contrainte imposée x à l'instant t_{f}. Elle précise en effet la plage des positions acceptables pour ce point, à l'instant futur objectif t_{f} correspondant par exemple à la fin du traitement avec l'appareil orthodontique actuel. Les bornes de cette plage correspondent à des situations dentaires extrêmes acceptables pour la valeur de *x* à l'instant t_{f}.

Dans un premier temps, l'orthodontiste peut choisir de ne pas imposer la plage P.

Les trois traitements orthodontiques potentiels T1, T2 et T3 peuvent par exemple différer par des valeurs pour la douleur ressentie, correspondant par exemple à trois réglages possibles de la tension de l'arc orthodontique. Par exemple un coefficient de douleur mesurant la douleur ressentie peut être par exemple de 200, 150 et 100 pour les traitements orthodontiques potentiels T1, T2 et T3, respectivement.

A partir de l'instant actuel tₐ, l'analyse statistique, effectuée pour chacun des instants futurs t₁, t₂ et t_{f} et, à chacun de ces instants futurs, pour les trois valeurs du coefficient de douleur, permet de prédire le traitement orthodontique en fonction du coefficient de douleur. On constate par exemple que si le patient accepte un coefficient de douleur de 200 (courbe T1), l'objectif pour le paramètre de contexte considéré sera atteint dès l'instant t₃. Il sera atteint à l'instant t₄ avec le coefficient de douleur de T2. La figure 2a montre que l'objectif de traitement ne sera pas atteint avec l'appareil orthodontique actuel si le patient actuel souhaite appliquer le coefficient de douleur de T3. En accord avec le patient actuel, l'orthodontiste peut alors choisir de rallonger la durée du traitement ou d'élargir la plage P ou de modifier le traitement orthodontique actuel, par exemple en changeant d'appareil orthodontique actuel, ce qui se traduira éventuellement par des coûts supplémentaires. La courbe T sur la figure 2a représente le traitement prévu initialement, à l'instant initial t₀. A l'instant actuel tₐ, on peut constater, en observant les dents du patient, un écart Δx important entre la position *xₐ* réelle et la position *x** prévue initialement à l'instant tₐ. Sans la prédiction grâce au système de contrôle selon l'invention, l'orthodontiste aurait probablement décidé de modifier le traitement, par exemple en changeant l'appareil orthodontique. Si l'appareil orthodontique actuel est celui correspondant à la courbe T2, il peut avantageusement constater que le retard sera rattrapé sans qu'il soit nécessaire de modifier le traitement.

Réciproquement, comme représenté sur la figure 2b, à l'instant actuel tₐ, l'orthodontiste peut constater, en observant les dents du patient, un écart Δx faible entre la position *xₐ* réelle et la position *x** prévue initialement à l'instant tₐ. Sans la prédiction grâce au système de contrôle selon l'invention, l'orthodontiste aurait probablement décidé de ne pas modifier le traitement. Si l'appareil orthodontique actuel est celui correspondant à la courbe T2, il peut avantageusement constater que l'écart va s'accroitre et que l'objectif ne sera pas atteint s'il ne modifie pas le traitement.

L'analyse statistique permet ainsi d'anticiper avec précision le comportement d'un appareil orthodontique en fonction de la valeur de ses paramètres et de la configuration des dents dans laquelle il est placé, mais aussi de simuler des traitements alternatifs, par exemple en modifiant les contraintes.

Les courbes correspondant aux traitements orthodontiques potentiels T1, T2 et T3 permettent également, à chaque instant futur, de visualiser l'écart entre la situation dentaire future prédite si un de ces traitements orthodontiques potentiels est appliqué et la situation dentaire constituant l'objectif audit instant futur (courbe T). En particulier, à l'instant t_{f}, la figure 2a permet de visualiser l'écart entre l'objectif pour la valeur du paramètre de contexte considéré, x_{f}, et les valeurs correspondantes x₁, x₂ et x₃ pour les situations dentaires futures prédites avec les contraintes associées aux traitements orthodontiques potentiels T1, T2 et T3.

L'orthodontiste peut aussi décider d'imposer la plage P comme contrainte à respecter. L'optimisation conduit alors à une représentation graphique comme celle de la figure 2c.

Dans un mode de réalisation, comme représenté sur la figure 2d, on détermine, à partir de l'instant initial t₀, par exemple en début de traitement, un traitement orthodontique potentiel dit « optimal » pour un paramètre, par exemple un paramètre de positionnement, et des traitements orthodontiques potentiels extrêmes correspondant à des limites minimale et maximale considérées comme acceptables pour ledit paramètre de positionnement. Ces traitements orthodontiques potentiels sont représentés par les courbes Tₒₚₜ, Tₘₐₓ et Tₘᵢₙ, respectivement, sur la figure 2d. Les courbes Tₘₐₓ et Tₘᵢₙ définissent ainsi une enveloppe fournissant, à tout instant jusqu'à l'instant futur objectif t_{f}, une tolérance par rapport à la courbe Tₒₚₜ relative au traitement optimal. A l'instant final t_{f}, par exemple en fin de traitement ou à un set-up intermédiaire, cette tolérance correspond à la plage P des positions finales acceptées. On appelle « biozone » la surface, hachurée sur les figures 2d et 2e, qui s'étend entre les deux courbes Tₘₐₓ et Tₘᵢₙ.

Sur la figure 2d, la courbe Tᵣ représente l'évolution réelle de la valeur du paramètre considéré, en l'occurrence la position x.

A tout instant, il est possible, très simplement, de vérifier si la position x est dans la biozone. Si c'est le cas, le traitement se déroule normalement. Sinon, il est nécessaire de corriger le traitement en conséquence.

Dans l'exemple de la figure 2d, la position x est sortie de la biozone à l'instant t₁, l'anomalie a été détectée à l'instant t₂ et le traitement a été modifié à l'instant t₃. La modification a permis de ramener la courbe Tᵣ dans la biozone, à partir de l'instant t₄.

La biozone est particulièrement un outil particulièrement efficace pour vérifier rapidement si un traitement se déroule comme prévu.

Dans l'exemple de la figure 2e, le procédé mis en œuvre par le système selon l'invention est utilisé hors traitement orthodontique, les dents du patient actuel étant normalement positionnées. La courbe optimale Tₒₚₜ correspond à une évolution « normale » pour le patient actuel, qui est une personne âgée. Les courbes Tₘₐₓ et Tₘᵢₙ définissent l'enveloppe de la biozone.

La position réelle *x* est surveillée. Chaque mesure de la position x est comparée, à l'instant correspondant, à la biozone. La position *x* est sortie de la biozone à l'instant t₁, l'anomalie a été détectée à l'instant t₂ et un traitement a été appliqué à partir de l'instant t₃. La modification a permis de ramener la courbe Tᵣ dans la biozone, à partir de l'instant t₄.

Comme cela apparaît clairement à présent, le procédé mis en œuvre par le système selon l'invention permet d'optimiser le traitement orthodontique, tout en améliorant l'information fournie au patient.

Bien entendu, l'invention n'est pas limitée aux modes de réalisation décrits ci-dessus et représentés.

En particulier, le patient n'est pas limité à un être humain. En particulier, un procédé de contrôle du positionnement de dents peut être utilisé pour un autre animal.

Le système de contrôle de 1 invention n'est pas limité au cadre d'un traitement orthodontique appliqué au patient actuel. Une prédiction de l'évolution de la position des dents est également possible alors que le patient ne suit aucun traitement, par exemple à des fins de surveillance.

## Revendications

1. Système de contrôle de la situation dentaire future d'un patient, dit « patient actuel », comportant :
- une base de données informatiques, sur laquelle des données, dites « données historiques », sont enregistrées, les données historiques étant relatives à plus de 1000 situations dentaires passées, dites « situations dentaires historiques », vécues chacune, à un instant, dit « instant historique », par un patient, dit « patient historique », l'ensemble des données historiques relatives à une situation dentaire historique comportant au moins:
- ledit instant historique ;
- des valeurs de paramètres de contexte audit instant historique, les paramètres de contexte comportant des paramètres de positionnement de dents dudit patient historique ;
- des moyens d'acquisition de données pour effectuer l'étape suivante :
acquisition, à un instant, dit « instant actuel », de données relatives à une situation dentaire vécue par ledit patient actuel, dite « situation dentaire actuelle », l'ensemble des données relatives à ladite situation dentaire actuelle, dites « données actuelles », comportant au moins:
- ledit instant actuel;
- des valeurs de paramètres de contexte audit instant actuel, les paramètres de contexte comportant des paramètres de positionnement de dents dudit patient actuel ;
- un programme d'ordinateur comprenant des instructions qui, lorsque le programme est exécuté par un ordinateur, conduisent celui-ci à mettre en œuvre les étapes suivantes :
∘ construction par analyse statistique, à partir desdites données historiques, d'un modèle prédictif permettant de prévoir comment la valeur d'un paramètre de positionnement « x » va évoluer en fonction de données d'entrée ;
∘ détermination, avec le modèle prédictif, les valeurs des paramètres de positionnement de dents du patient actuel à l'instant actuel étant desdites données d'entrée, de deux courbes (Tₘₐₓ, Tₘᵢₙ) fournissant l'évolution temporelle dudit paramètre de positionnement « x » de dents du patient actuel jusqu'à un instant futur objectif (t_{f}) et définissant des limites minimale et maximale considérées comme acceptables pour ledit paramètre de positionnement,
∘ représentation desdites courbes sur un même graphique,
lesdites deux courbes fournissent l'évolution temporelle dudit paramètre de positionnement « x » pour des premier et deuxième traitements orthodontiques potentiels conduisant, pour ledit paramètre de positionnement « x », à des situations dentaires extrêmes pour ledit instant futur objectif, une situation dentaire extrême correspondant à une limite minimale ou maximale pour ledit paramètre de positionnement des dents du patient actuel, ou
lesdites deux courbes délimitant, hors traitement orthodontique, une surface hors de laquelle la valeur du paramètre de positionnement « x » est une anomalie.

2. Système selon la revendication 1, dans lequel le programme d'ordinateur comprend des instructions qui lorsque le programme est exécuté par un ordinateur, conduisent celui-ci à déterminer, avec ledit modèle prédictif, une courbe optimale (Tₒₚₜ) représentant un traitement orthodontique potentiel optimal ou, hors traitement orthodontique, les dents du patient actuel étant normalement positionnées, une évolution normale dudit paramètre de positionnement de dents du patient actuel.

3. Système selon la revendication immédiatement précédente, dans lequel le critère d'optimisation pour la courbe optimale est choisi dans le groupe formé par un coefficient de douleur, un coût, un écart avec une valeur souhaitée pour un paramètre de positionnement, une durée, un nombre de rendez-vous chez l'orthodontiste, un nombre de gouttières, une probabilité de succès ou une combinaison de ces critères.

4. Système selon l'une quelconque des revendications précédentes, dans lequel les paramètres de contexte audit instant historique comportent:
- si le patient historique est muni d'un appareil orthodontique, dit « appareil orthodontique historique »,
∘ au moins un paramètre dudit appareil orthodontique historique relatif à la classe et/ou à la conformation de l'appareil orthodontique historique ; et/ou
∘ au moins un paramètre sur l'environnement du traitement orthodontique auquel la situation dentaire historique appartient, dit « traitement orthodontique historique », choisi parmi un coefficient de douleur, un coût, une durée, un nombre de rendez-vous chez un orthodontiste, et une probabilité de succès associé(s) audit traitement orthodontique historique, et/ou
- au moins un paramètre fonctionnel du patient historique ; et/ou
- au moins un paramètre anatomique du patient historique autre que les paramètres de positionnement de ses dents ; et/ou
- l'âge, le sexe et un identifiant dudit patient historique.

5. Système selon l'une quelconque des revendications précédentes, dans lequel les paramètres de contexte audit instant actuel comportent :
- si le patient actuel est muni d'un appareil orthodontique actuel,
∘ au moins un paramètre dudit appareil orthodontique actuel relatif à la classe et/ou à la conformation de l'appareil orthodontique actuel; et/ou
∘ au moins un paramètre sur l'environnement du traitement orthodontique actuel auquel la situation dentaire actuelle appartient, dit « traitement orthodontique actuel », choisi parmi un coefficient de douleur, un coût, une durée, un nombre de rendez-vous chez un orthodontiste et une probabilité de succès associé(s) audit traitement orthodontique actuel; et/ou
- au moins un paramètre fonctionnel du patient actuel ; et/ou
- au moins un paramètre anatomique du patient actuel autre que les paramètres de positionnement de ses dents ; et/ou
- l'âge, le sexe et un identifiant dudit patient actuel.

6. Système selon l'une quelconque des revendications précédentes, dans lequel le modèle prédictif respecte des contraintes ajustables par l'orthodontiste et/ou le patient.

7. Système selon la revendication immédiatement précédente, dans lequel les contraintes ajustables comprennent des contraintes pour lesquelles l'orthodontiste ou le patient actuel peuvent fixer des plages de variation, à l'instant actuel et/ou à un ou plusieurs instants futurs.

8. Système selon l'une quelconque des deux revendications immédiatement précédentes, dans lequel les contraintes ajustables comportent une plage de positions acceptables pour les dents du patient actuel à un instant futur, et/ou un coût maximal de traitement, et/ou un coefficient de douleur maximal au cours du traitement et/ou un coefficient de douleur moyen pendant un traitement orthodontique actuel.

9. Système selon l'une quelconque des revendications précédentes, dans lequel l'instant de départ du ou des traitements orthodontiques potentiels est l'instant actuel ou un instant initial correspondant au début d'un traitement orthodontique actuel avec un appareil orthodontique porté par le patient actuel.

## Patentansprüche

1. System zur Kontrolle der zukünftigen Zahnsituation eines Patienten, genannt "aktueller Patient", umfassend:
- eine Computerdatenbank, auf welcher Daten, sogenannte "historische Daten", gespeichert sind, wobei die historischen Daten sich auf mehr als 1.000 vergangene Zahnsituationen, sogenannte "historische Zahnsituationen", beziehen, die jeweils zu einem Zeitpunkt, sogenannter "historischer Zeitpunkt", von einem Patienten, sogenannter "historischer Patient", erlebt wurden, wobei die Gesamtheit der historischen Daten bezüglich einer historischen Zahnsituation mindestens umfasst:
- den besagten historischen Zeitpunkt;
- Kontextparameterwerte zu besagtem historischem Zeitpunkt, wobei die Kontextparameter Zahnpositionierungsparameter des besagten historischen Patienten umfassen;
- Datenerfassungsmittel zur Durchführung des folgenden Schrittes: Erfassung, zu einem Zeitpunkt, genannt "aktueller Zeitpunkt", von Daten bezüglich einer von besagtem aktuellem Patienten erlebten Zahnsituation, genannt "aktuelle Zahnsituation", wobei die Gesamtheit der Daten bezüglich besagter aktueller Zahnsituation, genannt "aktuelle Daten", mindestens umfasst:
- den besagten aktuellen Zeitpunkt;
- Kontextparameterwerte zu besagtem aktuellem Zeitpunkt, wobei die Kontextparameter Zahnpositionierungsparameter des besagten aktuellen Patienten umfassen;
- ein Computerprogramm, das Anweisungen umfasst, die, wenn das Programm von einem Computer ausgeführt wird, diesen dazu veranlassen, die folgenden Schritte durchzuführen:
∘ Aufbau eines prädiktiven Modells durch statistische Analyse der besagten historischen Daten, das es ermöglicht vorherzusagen, wie sich der Wert eines Positionierungsparameters "x" in Abhängigkeit von Eingangsdaten entwickeln wird;
∘ Bestimmung, mit dem prädiktiven Modell, wobei die Werte der Zahnpositionierungsparameter des aktuellen Patienten zum aktuellen Zeitpunkt besagte Eingangsdaten sind, von zwei Kurven (Tₘₐₓ, Tₘᵢₙ), die die zeitliche Entwicklung des besagten Zahnpositionierungsparameters "x" des aktuellen Patienten bis zu einem zukünftigen Zielzeitpunkt (t_{f}) liefern und minimale und maximale Grenzen definieren, die für den besagten Positionierungsparameter als akzeptabel betrachtet werden,
∘ Darstellung der besagten Kurven in einem gleichen Diagramm,
wobei die besagten zwei Kurven die zeitliche Entwicklung des besagten Positionierungsparameters "x" für potentielle erste und zweite kieferorthopädische Behandlungen liefern, die für den besagten Positionierungsparameter "x" zu extremen Zahnsituationen für den besagten zukünftigen Zielzeitpunkt führen, wobei eine extreme Zahnsituation einer minimalen oder maximalen Grenze für den besagten Zahnpositionierungsparameter des aktuellen Patienten entspricht, oder
wobei die besagten zwei Kurven außerhalb einer kieferorthopädischen Behandlung eine Fläche abgrenzen, außerhalb welcher der Wert des Positionierungsparameters "x" eine Anomalie darstellt.

2. System nach Anspruch 1, wobei das Computerprogramm Anweisungen umfasst, die, wenn das Programm von einem Computer ausgeführt wird, diesen dazu veranlassen, mit dem prädiktiven Modell eine optimale Kurve (Tₒₚₜ) zu bestimmen, die eine optimale potentielle kieferorthopädische Behandlung darstellt oder, ohne kieferorthopädische Behandlung, wenn die Zähne des aktuellen Patienten normal positioniert sind, eine normale Entwicklung des Zahnpositionierungsparameters des aktuellen Patienten darstellt.

3. System nach dem unmittelbar vorstehenden Anspruch, wobei das Optimierungskriterium für die optimale Kurve aus der Gruppe gewählt wird, die aus einem Schmerzkoeffizienten, Kosten, einer Abweichung von einem gewünschten Wert für einen Positionierungsparameter, einer Dauer, einer Anzahl von Terminen beim Kieferorthopäden, einer Anzahl von Schienen, einer Erfolgswahrscheinlichkeit oder einer Kombination dieser Kriterien gebildet wird.

4. System nach einem der vorstehenden Ansprüche, wobei die Kontextparameter zu besagtem historischem Zeitpunkt umfassen:
- wenn der historische Patient mit einer kieferorthopädischen Vorrichtung versehen ist, diese "historische kieferorthopädische Vorrichtung",
∘ mindestens einen Parameter der besagten historischen kieferorthopädischen Vorrichtung bezüglich der Klasse und/oder der Konformation der historischen kieferorthopädischen Vorrichtung; und/oder
∘ mindestens einen Parameter zur Umgebung der kieferorthopädischen Behandlung, zu der die historische Zahnsituation gehört, genannt "historische kieferorthopädische Behandlung", ausgewählt aus einem Schmerzkoeffizienten, Kosten, einer Dauer, einer Anzahl von Terminen bei einem Kieferorthopäden und einer Erfolgswahrscheinlichkeit, die der historischen kieferorthopädischen Behandlung zugeordnet ist/sind, und/oder
- mindestens einen funktionellen Parameter des historischen Patienten; und/oder
- mindestens einen anatomischen Parameter des historischen Patienten, der von den Positionierungsparametern seiner Zähne verschieden ist; und/oder
- das Alter, das Geschlecht und eine Kennung des besagten historischen Patienten.

5. System nach einem der vorstehenden Ansprüche, wobei die Kontextparameter zu besagtem aktuellem Zeitpunkt umfassen:
- wenn der aktuelle Patient mit einer aktuellen kieferorthopädischen Vorrichtung versehen ist,
∘ mindestens einen Parameter der besagten aktuellen kieferorthopädischen Vorrichtung bezüglich der Klasse und/oder der Konformation der aktuellen kieferorthopädischen Vorrichtung; und/oder
∘ mindestens einen Parameter zur Umgebung der aktuellen kieferorthopädischen Behandlung, zu der die aktuelle Zahnsituation gehört, genannt "aktuelle kieferorthopädische Behandlung", ausgewählt aus einem Schmerzkoeffizienten, Kosten, einer Dauer, einer Anzahl von Terminen bei einem Kieferorthopäden und einer Erfolgswahrscheinlichkeit, die der besagten aktuellen kieferorthopädischen Behandlung zugeordnet ist/sind; und/oder
- mindestens einen funktionellen Parameter des aktuellen Patienten; und/oder
- mindestens einen anatomischen Parameter des aktuellen Patienten, der von den Positionierungsparametern seiner Zähne verschieden ist; und/oder
- das Alter, das Geschlecht und eine Kennung des besagten aktuellen Patienten.

6. System nach einem der vorstehenden Ansprüche, wobei das prädiktive Modell durch den Kieferorthopäden und/oder den Patienten einstellbare Beschränkungen einhält.

7. System nach dem unmittelbar vorstehenden Anspruch, wobei die anpassbaren Beschränkungen Beschränkungen umfassen, für welche der Kieferorthopäde oder der aktuelle Patient Variationsbereiche zum aktuellen Zeitpunkt und/oder zu einem oder mehreren zukünftigen Zeitpunkten festlegen können.

8. System nach einem der beiden unmittelbar vorstehenden Ansprüche, wobei die anpassbaren Beschränkungen einen Bereich akzeptabler Positionen für die Zähne des aktuellen Patienten zu einem zukünftigen Zeitpunkt und/oder maximale Behandlungskosten und/oder einen maximalen Schmerzkoeffizienten während der Behandlung und/oder einen durchschnittlichen Schmerzkoeffizienten während einer aktuellen kieferorthopädischen Behandlung umfassen.

9. System nach einem der vorstehenden Ansprüche, wobei der Startzeitpunkt der potentiellen kieferorthopädischen Behandlung oder Behandlungen der aktuelle Zeitpunkt oder ein Anfangszeitpunkt ist, der dem Beginn einer aktuellen kieferorthopädischen Behandlung mit einer kieferorthopädischen Vorrichtung entspricht, die von dem aktuellen Patienten getragen wird.

## Claims

1. System for monitoring the future dental situation of a patient, referred to as a "current patient", the system including:
- a computer database on which data, referred to as "historical data", are recorded, the historical data relating to more than 1000 past dental situations, referred to as "historical dental situations", each experienced at a time, referred to as a "historical time", by a patient, referred to as a "historical patient", the set of the historical data relating to a historical dental situation including at least:
- said historical time;
- values of context parameters at said historical time, the context parameters including teeth positioning parameters for said historical patient;
- data acquisition means for carrying out the following step:
acquiring at a time, referred to as a "current time", data relating to a dental situation experienced by said current patient, referred to as a "current dental situation", the set of the data relating to said current dental situation, referred to as "current data", including at least:
- said current time;
- values of context parameters at said current time, the context parameters including teeth positioning parameters for said current patient;
- a computer program comprising instructions which, when the program is executed by a computer, cause said computer to implement the following steps:
∘ constructing, using statistical analysis and on the basis of said historical data, a predictive model for forecasting how the value of a positioning parameter "x" will change as a function of input data;
∘ determining with the predictive model, with the values of the teeth positioning parameters for the current patient at the current time being said input data, two curves (Tₘₐₓ, Tₘᵢₙ) that provide the change over time of said teeth positioning parameter "x" for the current patient up to a target future time (t_{f}) and define minimum and maximum limits considered acceptable for said positioning parameter,
∘ representing said curves on the same graph,
said two curves provide the change over time of said positioning parameter "x" for a first and a second potential orthodontic treatment leading, for said positioning parameter "x", to extreme dental situations for said target future time, an extreme dental situation corresponding to a minimum or maximum limit for said positioning parameter for the teeth of the current patient, or
said two curves delimiting, excluding orthodontic treatment, a surface outside which the value of the positioning parameter "x" is an anomaly.

2. System according to claim 1, wherein the computer program comprises instructions which, when the program is executed by a computer, cause said computer to determine with said predictive model an optimal curve (Tₒₚₜ) that represents an optimal potential orthodontic treatment or, excluding orthodontic treatment, with the teeth of the current patient normally positioned, a normal change in said teeth positioning parameter for the current patient.

3. System according to the immediately preceding claim, wherein the optimization criterion for the optimal curve is selected from the group formed of a pain coefficient, a cost, a deviation from a desired value for a positioning parameter, a duration, a number of orthodontist appointments, a number of aligners, a probability of success, or a combination of these criteria.

4. System according to any one of the preceding claims, wherein the context parameters at said historical time include:
- if the historical patient has an orthodontic device, referred to as a "historical orthodontic device",
∘ at least one parameter for said historical orthodontic device relating to the class and/or conformation of the historical orthodontic device; and/or
∘ at least one parameter concerning the environment of the orthodontic treatment to which the historical dental situation belongs, referred to as a "historical orthodontic treatment", selected from a pain coefficient, a cost, a duration, a number of orthodontist appointments, and a probability of success, associated with said historical orthodontic treatment, and/or
- at least one functional parameter for the historical patient; and/or
- at least one anatomical parameter for the historical patient other than the positioning parameters for their teeth; and/or
- the age, the gender, and an identifier of said historical patient.

5. System according to any one of the preceding claims, wherein the context parameters at said current time include:
- if the current patient has a current orthodontic device,
∘ at least one parameter for said current orthodontic device relating to the class and/or conformation of the current orthodontic device; and/or
∘ at least one parameter concerning the environment of the current orthodontic treatment to which the current dental situation belongs, referred to as a "current orthodontic treatment", selected from a pain coefficient, a cost, a duration, a number of orthodontist appointments, and a probability of success, associated with said current orthodontic treatment; and/or
- at least one functional parameter for the current patient; and/or
- at least one anatomical parameter for the current patient other than the positioning parameters for their teeth; and/or
- the age, the gender, and an identifier of said current patient.

6. System according to any one of the preceding claims, wherein the predictive model respects constraints adjustable by the orthodontist and/or the patient.

7. System according to the immediately preceding claim, wherein the adjustable constraints comprise constraints for which the orthodontist or the current patient can set variation ranges at the current time and/or at one or more future times.

8. System according to either one of the two immediately preceding claims, wherein the adjustable constraints include a range of acceptable positions for the teeth of the current patient at a future time, and/or a maximum treatment cost, and/or a maximum pain coefficient during the treatment and/or an average pain coefficient during a current orthodontic treatment.

9. System according to any one of the preceding claims, wherein the starting time of the potential orthodontic treatment or treatments is the current time or an initial time corresponding to the start of a current orthodontic treatment with an orthodontic device worn by the current patient.
